(19) Europäisches Patentamt
European Patent Office
Office européen des brevets

(11) EP 1 153 918 B1

(12) EUROPÄISCHE PATENTSCHRIFT

(45) Veröffentlichungstag und Bekanntmachung des Hinweises auf die Patenterteilung:
**08.09.2004 Patentblatt 2004/37**

(51) Int Cl.[7]: **C07D 211/02**, C07D 211/60, C07D 471/04

(21) Anmeldenummer: **01109476.0**

(22) Anmeldetag: **25.04.2001**

# (54) Verfahren zur Herstellung von Piperidinen durch katalytische Hydrierung von Pyridinen

Process for the preparation of piperidines from pyridines using catalytic hydrogenation

Procédé de préparation des piperidines par hydrogénation catalytique des pyridines

(84) Benannte Vertragsstaaten:
**AT BE CH CY DE DK ES FI FR GB GR IE IT LI LU MC NL PT SE TR**

(30) Priorität: **08.05.2000 DE 10022369**

(43) Veröffentlichungstag der Anmeldung:
**14.11.2001 Patentblatt 2001/46**

(73) Patentinhaber: **Bayer Chemicals AG**
**51368 Leverkusen (DE)**

(72) Erfinder:
- **Giffels, Guido, Dr.**
**53177 Bonn (DE)**
- **Diehl, Herbert, Dr.**
**51373 Leverkusen (DE)**
- **Martin, Georg, Dr.**
**40764 Langenfeld (DE)**
- **Frohn, Lutz, Dr.**
**40699 Erkrath (DE)**
- **Hammerschmidt, Erich, Dr.**
**51467 Bergisch Gladbach (DE)**

(56) Entgegenhaltungen:
**EP-A- 0 350 733** **US-A- 5 942 104**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 153 918 B1

**Beschreibung**

Die vorliegende Erfindung betrifft ein verbessertes Verfahren zur Herstellung von Piperidinen durch Kernhydrierung der entsprechenden Pyridine.

Piperidine sind Zwischenprodukte für die Herstellung pharmazeutischer Wirkstoffe (siehe z.B. EP-A 603 887 und EP-A 350 733, insbesondere Seiten 2 und 17). Sie müssen deshalb in möglichst reiner Form verfügbar sein.

Aktivierte Piperidine werden üblicherweise mit Palladiumkatalysatoren in alkoholischen Lösungsmitteln wie Methanol, Ethanol, Isopropanol oder Ethylenglykolmonomethylether zu den entsprechenden Piperidinen hydriert (siehe Heterogenous Catalysis for the Synthetics Chemist, New York 1996, 17. Kapitel, S. 421 bis 424 und EP-A 350 733, insbesondere Seiten 65 und 66). Die Aufarbeitung des Reaktionsgemisches erfolgt im Allgemeinen durch Abtrennen des Katalysators und des Lösungsmittels. Es resultieren dann Piperidine, die noch weiter gereinigt werden müssen, z.B. durch Kristallisation, Destillation oder mittels Chromatographie. Auf diese Weise werden unerwünschte, in den Piperidinen enthaltenen Nebenprodukte entfernt. Neben dem Aufwand, der mit einer solchen Reinigung verbunden ist, ist auch der bei der weiteren Reinigung eintretende Produktverlust nachteilig, zumal das Lösungsmittel aufgrund der dann darin enthaltenen Verunreinigungen nur bedingt zurückgeführt werden kann.

Aus US-5 942 014 (Beispiel 4) ist ein Verfahren zur Hydrierung eines Pyridinis in einem Gemisch aus $\frac{1}{3}$ Toluol und $\frac{2}{3}$ Ethylacetat bekannt.

Es besteht also noch ein Bedürfnis nach einem Verfahren zur Herstellung von Piperidinen, bei dem keine weitere Reinigung des aus dem Reaktionsgemisch isolierten Produktes nötig ist.

Es wurde nun ein Verfahren zur Herstellung von Piperidinen durch katalytische Hydrierung von aktivierten Pyridinen in Gegenwart von Palladiumkatalysatoren gefunden, das dadurch gekennzeichnet ist, dass man als Palladiumkatalysatoren Palladium-auf-Kohle und als Lösungsmittel aromatische Kohlenwasserstoffe einsetzt.

Als aktivierte Pyridine werden in das erfindungsgemäße Verfahren solche der Formel (I) eingesetzt

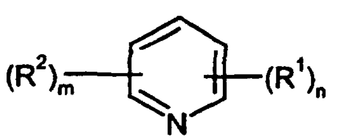 (I),

in der

$R^1$ für $COOR^3$-, $CONH_2$-, $CO-NH-COR^3$- oder COOH-Gruppen oder zwei benachbarte $R^1$-Gruppen gemeinsam für eine $CO-NR^4-CO$-Gruppe stehen,

$R^2$ für lineares oder verzweigtes $C_1$-$C_{20}$-Alkyl steht,

$R^3$ für lineares oder verzweigtes $C_1$-$C_6$-Alkyl, Phenyl oder Benzyl steht,

$R^4$ für Wasserstoff, für lineares oder verzweigtes $C_1$-$C_6$-Alkyl, Phenyl oder Benzyl steht,

n für 1 oder 2 steht und

m für Null, 1 oder 2 steht.

Wenn n für 2 steht können zwei gleich oder zwei verschiedene Reste $R^1$ vorhanden sein, ebenso können, wenn m für 2 steht, zwei gleiche oder verschiedene Reste $R^2$ vorhanden sein.

Vorzugsweise steht $R^1$ für $COO-C_1$-$C_4$-Alkyl oder zwei benachbarte $R^1$-Gruppen gemeinsam für eine CO-N(Benzyl)-CO-Gruppe, $R^2$ für $C_1$-$C_4$-Alkyl, n für 1 oder 2 und m für Null oder 1.

Wenn man in das erfindungsgemäße Verfahren aktivierte Pyridine der Formel (I) einsetzt, erhält man die entsprechenden Piperidine der Formel (II)

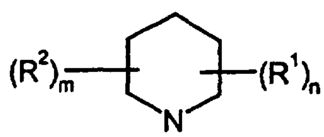 (II),

in der die verwendeten Symbole die bei Formel (I) angegebene Bedeutung haben.

Bei den erfindungsgemäß einzusetzenden Palladium-auf-Kohle-Katalysatoren kann es sich beispielsweise um solche handeln, die 1 bis 10 Gew.-% Palladium auf einer beliebigen Kohle enthalten. Vorzugsweise enthalten die Katalysatoren 2 bis 8 Gew.-% Palladium. Geeignete Katalysatoren sind im Handel erhältlich.

Man kann z.B. soviel Katalysator einsetzen, dass 0,5 bis 30 mMol Palladium, bezogen auf 1 Mol aktiviertes Pyridin, vorliegen. Vorzugsweise liegt diese Menge bei 2 bis 15 mMol.

Als aromatische Kohlenwasserstoffe kommen z.B. Benzol, Toluol, Xylole und sonstige Alkylaromaten infrage. Bevorzugt ist Toluol. Bezogen auf 1 mol aktiviertes Pyridin kann man z.B. 50 bis 5000 g aromatische Kohlenwasserstoff (auch in Form von Gemischen) einsetzen.

Die erfindungsgemäße katalytische Hydrierung kann man z.B. bei Temperaturen im Bereich 20 bis 200°C durchführen. Bevorzugt sind Temperaturen im Bereich 50 bis 150°C, insbesondere solche im Bereich 60 bis 100°C. Als Drücke kommen z.B. solche im Bereich 1 bis 200 bar infrage. Bevorzugt sind Drücke im

Bereich 3 bis 150 bar, insbesondere solche im Bereich 5 bis 60 bar.

Die gleichzeitige Anwendung von Temperaturen und Drücken nahe der Obergrenze ist vorteilhafterweise zu vermeiden, weil sonst die Gefahr der Mitreduktion des Lösungsmittels besteht.

Nach Durchführung der katalytischen Hydrierung kann man das vorliegende Reaktionsgemisch z.B. aufarbeiten, indem man den Katalysator abtrennt, z.B. durch Filtration und danach den aromatischen Kohlenwasserstoff entfernt, z.B. durch Destillation, gegebenenfalls bei vermindertem Druck. Sowohl der abgetrennte Katalysator, als auch der abgetrennte aromatische Kohlenwasserstoff können recyclisiert werden. Gegebenenfalls kann recyclisiertem Katalysator und recyclisiertem aromatischen Kohlenwasserstoff frischer Katalysator bzw. frischer aromatischer Kohlenwasserstoff hinzugefügt werden.

Nach Abtrennung des Katalysators und des aromatischen Kohlenwasserstoffs hinterbleiben die hergestellten Piperidine im Allgemeinen in Reinheiten von über 98 %. Eine weitere Reinigung ist deshalb nicht nötig. Wie aus den Vergleichsbeispielen hervorgeht, werden beim üblichen Arbeiten mit alkoholischen Lösungsmitteln ohne weitere Reinigung nur Piperidine mit Reinheiten um 94 % erhalten. Für die Weiterverwendung der Piperidine als Zwischenprodukte für Pharmazeutika ist die erfindungsgemäß erzielbare Reinheit von entscheidender Bedeutung.

Es ist ausgesprochen überraschend, dass man gemäß der vorliegenden Erfindung ein so vorteilhaftes Verfahren aufgefunden hat, denn es war aus der bekannten Literatur nicht zu erwarten, dass bei der heterogenen Katalyse mit Palladium-auf-Kohle Katalysatoren in unpolaren Lösungsmittel hohe Ausbeuten und Selektivitäten erzielbar sind. Weiterhin war zu erwarten, dass nicht nur die eingesetzten Pyridine, sondern auch die als Lösungsmittel zu verwendenden aromatischen Kohlenwasserstoffe hydriert werden und somit nicht nur in verunreinigte Produkte, sondern auch noch zusätzlich Lösungsmittelverluste auftreten. Letzteres ist jedoch nur in sehr geringem Maß der Fall.

## Beispiele

In einem 0,7 l-Rührautoklaven mit Rührer, Thermofühler und Steigrohr wurden 163,7 g Pyridin-2,3-dicarbonsäure-N-benzylimid und 6,6 g 5 Gew.-% Palladium-auf-Kohle in 256,7 g Toluol suspendiert. Der Autoklav wurde zweimal mit Stickstoff und anschließend zweimal mit Wasserstoff gespült. Anschließend heizte man unter einem Wasserstoffdruck von 5 bar auf 80°C und erhöhte dann den Wasserstoffdruck schrittweise auf 50 bar, so dass die Reaktionstemperatur von 80°C eingehalten werden konnte. Nach Erreichen von 50 bar wurde bei 80°C noch 10 Stunden nachgerührt. Danach wurde abgekühlt, der Katalysator aus dem Reaktionsgemisch durch Filtration abgetrennt und Toluol am Rotationsverdampfer unter vermindertem Druck entfernt. Man erhielt 170 g Piperidin-2,3-dicarbonsäure-N-benzylimid in einer Reinheit von 98,8 % (Flächenprozent nach GC).

## Beispiel 2

Es wurde gearbeitet wie in Beispiel 1, jedoch wurden 181,7 g Pyridin-2,3-dicarbonsäure-N-benzylimid eingesetzt und bei einem Druck von 10 bar hydriert. Man erhielt 183,2 g Piperidin-2,3-dicarbonsäure-N-benzylimid in einer Reinheit von 98,2 % (Flächenprozent nach GC).

## Beispiel 3

In einem 1,3 l-Rührautoklaven mit Rührer, Thermofühler und Steigrohr wurden 666 g Pyridin-2,3-dicarbonsäure-N-benzylimid und 28,2 g 5 Gew.-% Palladium-auf-Kohle in 959 g Toluol suspendiert. Der Autoklav wurde danach gespült und mit Wasserstoff beaufschlagt und auf eine Reaktionstemperatur von 80°C gebracht, wie im Beispiel 1 beschrieben. Nach einer Aufarbeitung des Reaktionsgemisches wie in Beispiel 1 beschrieben, erhielt man 618 g Piperidin-2,3-dicarbonsäure-N-benzylimid in einer Reinheit von 98,7 % (Flächenprozent nach GC).

## Beispiel 4

In einem 0,3 l-Rührautoklaven mit Rührer und Thermofühler wurden 19,52 g Pyridin-2,3-dicarbonsäuredimethylester und 2,25 g 5 Gew.-% Palladium-auf-Kohle in 131 g Toluol vorgelegt. Der Autoklav wurde zweimal mit Stickstoff und anschließend zweimal mit Wasserstoff gespült. Dann wurde auf 80°C aufgeheizt und nach Erreichen dieser Temperatur 10 bar Wasserstoff aufgedrückt und unter diesen Bedingungen 4 Stunden lang hydriert. Nach Abkühlung des Reaktionsgemisches wurde der Katalysator durch Filtration abgetrennt und Toluol am Rotationsverdampfer unter vermindertem Druck entfernt. Man erhielt 19,13 g cis-Piperidin-2,3-dicarbonsäuredimethylester in einer Reinheit von 98,7 % (Flächenprozent nach GC).

**Vergleichsbeispiel 1** (Isopropanol als Lösungsmittel)

Es wurde gearbeitet wie in Beispiel 2, jedoch wurden statt Toluol 261,7 g Isopropanol verwendet. Es wurden 185,7 g Piperidin-2,3-dicarbonsäure-N-benzylimid in einer Reinheit von 94 % (Flächenprozent nach GC) erhalten.

**Vergleichsbeispiel 2** (Ethylenglykolmonomethylether als Lösungsmittel)

Es wurde gearbeitet wie in Beispiel 1, jedoch wurde statt Toluol die gleiche Menge Ethylenglykolmonomethylether eingesetzt. Man erhielt 171,1 g Piperidin-2,3-dicarbonsäure-N-benzylimid in einer Reinheit von 94,8 % (Flächenprozent nach GC).

## Patentansprüche

1. Verfahren zur Herstellung von Piperidinen durch katalytische Hydrierung von Pyridinen der Formel (I),

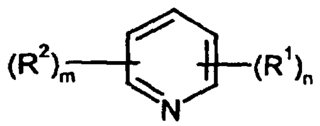

(I),

in der

$R^1$ für $COOR^3$-, $CONH_2$-, $CO-NH-COR^3$- oder COOH-Gruppen oder zwei benachbarte $R^1$-Gruppen gemeinsam für eine $CO-NR^4-CO$-Gruppe stehen,

$R^2$ für lineares oder verzweigtes $C_1$-$C_{20}$-Alkyl oder Halogen steht,

$R^3$ für lineares oder verzweigtes $C_1$-$C_6$-Alkyl, Phenyl oder Benzyl steht,

$R^4$ für Wasserstoff, für lineares oder verzweigtes $C_1$-$C_6$-Alkyl, Phenyl oder Benzyl steht,

n für 1 oder 2 steht und

m für Null, 1 oder 2 steht

in Gegenwart von Palladium-auf-Kohle-Katalysatoren, **dadurch gekennzeichnet, dass** man als Lösungsmittel aromatische Kohlenwasserstoffe einsetzt
und die entsprechenden Piperidine der Formel (II) erhält

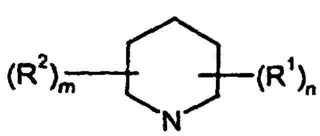

(II),

in der die verwendeten Symbole die bei Formel (I) angegebene Bedeutung haben.

2. Verfahren nach Anspruch 1, **dadurch gekennzeichnet, dass** in den Formeln (I) und (II) $R^1$ für $COO-C_1$-$C_4$-Alkyl oder zwei benachbarte $R^1$-Gruppen gemeinsam für eine CO-N(Benzyl)-CO-Gruppe, $R^2$ für $C_1$-$C_4$-Alkyl, n für 1 oder 2 und m für Null oder 1 stehen.

3. Verfahren nach Ansprüchen 1 und 2, **dadurch gekennzeichnet, dass** man Palladium-auf-Kohle-Katalysatoren einsetzt, die 1 bis 10 Gew.-% Palladium enthalten.

4. Verfahren nach Ansprüchen 1 bis 3, **dadurch gekennzeichnet, dass** man soviel Katalysator einsetzt, dass 0,5 bis 30 mMol Palladium, bezogen auf 1 Mol eingesetztes Pyridin, vorliegt.

5. Verfahren nach Ansprüchen 1 bis 4, **dadurch gekennzeichnet, dass** man als aromatische Kohlenwasserstoffe Benzol, Toluol, Xylol oder andere Alkylaromaten einsetzt.

6. Verfahren nach Ansprüchen 1 bis 5, **dadurch gekennzeichnet, dass** man es bei Temperaturen im Bereich 20 bis 200°C durchführt.

7. Verfahren nach Ansprüchen 1 bis 6, **dadurch gekennzeichnet, dass** man es bei Drücken im Bereich von 1 bis 200 bar durchführt.

## Claims

1. Process for preparing piperidines by catalytically hydrogenating pyridines of formula (I)

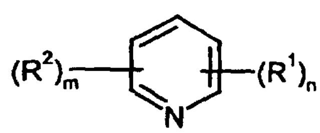

(I),

wherein
$R^1$ represents $COOR^3$, $CONH_2$, $CO-NH-COR^3$ or COOH groups, or two adjacent $R^1$ groups together represent a $CO-NR^4-CO$ group,
$R^2$ represents linear or branched $C_1$-$C_{20}$ alkyl or halogen,
$R^3$ represents linear or branched $C_1$-$C_6$ alkyl, phenyl or benzyl,
$R^4$ represents hydrogen, linear or branched $C_1$-$C_6$ alkyl, phenyl or benzyl,
n represents 1 or 2, and
m represents zero, 1 or 2,
in the presence of palladium-on-carbon catalysts, **characterised in that** aromatic hydrocarbons are used as solvents,
and the corresponding piperidines of formula (II) are obtained

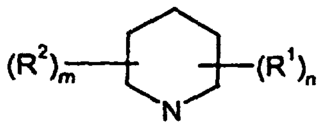

(II),

wherein the symbols used have the meaning as defined in formula (I).

2. Process according to claim 1, **characterised in that**, in formulae (I) and (II), $R^1$ represents COO-$C_1$-$C_4$ alkyl, or two adjacent $R^1$ groups together represent a CO-N(benzyl)-CO group, $R^2$ represents $C_1$-$C_4$ alkyl, n represents 1 or 2, and m represents zero or 1.

3. Process according to either claim 1 or claim 2, **characterised in that** palladium-on-carbon catalysts that contain from 1 to 10 % by weight palladium are used.

4. Process according to claims 1 to 3, **characterised in that** an amount of catalyst is used such that there are 0.5 to 30 mmol palladium per mol of pyridine used.

5. Process according to claims I to 4, **characterised in that** the aromatic hydrocarbons used are benzene, toluene, xylene or other alkyl aromatics.

6. Process according to claims 1 to 5, **characterised in that** it is carried out at temperatures in the range from 20 to 200 °C.

7. Process according to claims 1 to 6, **characterised in that** it is carried out at pressures in the range from 1 to 200 bar.

**Revendications**

1. Procédé de préparation de pipéridines par hydrogénation catalytique de pyridines de la formule (I)

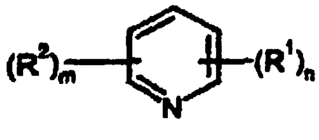

(I),

dans laquelle

$R^1$ représente des groupes $COOR^3$, $CONH_2$, CO-NH-$COR^3$ ou COOH ou deux groupes $R^1$ voisins représentent ensemble un groupe CO-$NR^4$-CO,

$R^2$ correspond à un alkyle en $C_1$-$C_{20}$ linéaire ou ramifié ou à un halogène,

$R^3$ équivaut à un alkyle en $C_1$-$C_6$ linéaire ou ramifié, à un phényle ou à un benzyle,

$R^4$ représente l'hydrogène, un alkyle en $C_1$-$C_6$ linéaire ou ramifié, un phényle ou un benzyle

n est égal à 1 ou à 2 et

m équivaut à 0, à 1 ou à 2

en présence de catalyseurs à base de palladium sur charbon, **caractérisé en ce que** l'on utilise comme solvants des hydrocarbures aromatiques,
et on obtient les pipéridines correspondantes de la formule (II)

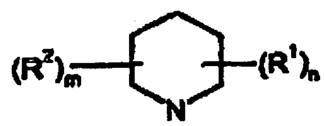

(II),

dans laquelle les symboles utilisés possèdent la signification indiquée pour la formule (I).

2. Procédé selon la revendication 1, **caractérisé en ce que** dans les formules (I) et (II), $R^1$ représente un groupe COO-alkyle en $C_1$-$C_4$ ou deux groupes $R^1$ voisins représentent ensemble un groupe CO-N (benzyl)-CO, $R^2$ correspond à un alkyle en $C_1$-$C_4$, n est égal à 1 ou à 2 et m équivaut à 0 ou à 1.

3. Procédé selon la revendication 1 ou 2, **caractérisé en ce que** l'on utilise des catalyseurs à base de palladium sur charbon, qui contiennent 1 à 10 % en poids de palladium.

4. Procédé selon les revendications 1 à 3, **caractérisé en ce que** l'on utilise une proportion de catalyseur telle qu'il y ait 0,5 à 30 mmol de palladium pour 1 mol de pyridine utilisée

5. Procédé selon les revendications 1 à 4, **caractérisé en ce que** l'on utilise comme hydrocarbures aromatiques, du benzène, du toluène, du xylène ou d'autres composés alkylaromatiques.

6. Procédé selon les revendications 1 à 5, **caractérisé en ce que** l'on opère celui-ci à des températures comprises dans l'intervalle de 20 à 200 °C.

7. Procédé selon les revendications 1 à 6, **caractérisé en ce que** l'on opère celui-ci à des pressions comprises dans l'intervalle de 1 à 200 bar.